# EUROPÄISCHE PATENTSCHRIFT

(11) **EP 0 608 401 B1**
(45) Veröffentlichungstag und Bekanntmachung des Hinweises auf die Patenterteilung: **15.01.1997**
(21) Anmeldenummer: 93917428.0
(22) Anmeldetag: 02.08.1993
(51) Int. Cl.: B01L 7/00, C12M 1/00

(54) **INKUBATOR**
INCUBATOR
INCUBATEUR

(30) Priorität: 18.08.1992 AT 1662/92
(43) Veröffentlichungstag der Anmeldung: 03.08.1994
(73) Patentinhaber: ROBOCON LABOR- UND INDUSTRIEROBOTER GES.M.B.H., 1110 Wien (AT)
(72) Erfinder: PIELER, Christian,Dr., A-1190 Wien (AT); LEICHTFRIED, Franz, Ernst,Dr., A-1080 Wien (AT)
(74) Vertreter: Pinter, Rudolf, Dipl.-Ing.
(86) Internationale Anmeldenummer: AT9300125
(87) Internationale Veröffentlichungsnummer: WO9404273

(56) Entgegenhaltungen:
- EP-A- 0 195 088
- EP-A- 0 238 313
- EP-A- 0 429 030
- WO-A-87/00087
- WO-A-93/08914

## Beschreibung

### Technisches Gebiet:

Die Erfindung betrifft einen Inkubator zur Aufnahme von Probenbehältern, mit einem frontseitig öffenbaren Gehäuse und einem in diesem stapelartig, beabstandet übereinander angeordneten Satz von Trageböden für jeweils mehrere bedarfsweise einzeln entfernbare Probenbehälter.

### Stand der Technik:

Derartige Geräte sind in verschiedensten Ausführungen bekannt, wobei entsprechend dem jeweiligen Anwendungsfall zumeist zumindest die Innentemperatur regelbar bzw. auf einen bestimmten Wert einstellbar ist. Speziell bei den heutzutage immer häufiger werdenden Anwendungsgebieten beispielsweise in der klinischen Diagnostik, in Qualitätskontrollabors der pharmazeutischen Industrie, in der pharmazeutischen Forschung und Entwicklung sowie in der Biotechnologie werden aber auch höhere Anforderungen an die Qualität der Innenatmosphäre gestellt, die dementsprechend auch beispielsweise hinsichtlich ihrer Zusammensetzung, Feuchtigkeit bzw. des Anteils an verschiedenen Komponenten regelbar ist.

Im Zusammenhang mit den angesprochenen Anwendungsgebieten besteht seit der Zeit des immer häufiger werdenden Einsatzes von Handhabungsrobotern das Bedürfnis, derartige Automaten auch für die dabei sich immer wiederholenden Handhabungsschritte zu verwenden, was aber nicht zuletzt auch im Hinblick auf die verwendeten Inkubatoren eine Reihe von Problemen aufwirft, die bisher mit den bekannten Geräten der genannten Art nur unzufriedenstellend gelöst werden konnten. So macht beispielsweise der erforderliche Zugriff zum Innenraum des Inkubators für die Beschickung mit den Probenbehältern bzw. die zwischenzeitliche Entnahme für Manipulationen am Inhalt der einzelnen Probenbehälter insoferne Schwierigkeiten, als dieser einerseits über eine Bedienperson von Hand und andererseits automatisch über den Handhabungsroboter erfolgen können soll, wobei aber auf alle Fälle jedes Unfallrisiko ausgeschaltet sein muß. Da die anfängliche Beschickung des Inkubators beispielsweise am Beginn eines Testzyklus üblicherweise von Hand erfolgt, muß eine einfache, schnelle und exakte Positionierung der einzelnen Probenbehälter relativ zum Handhabungsroboter möglich sein, was aber bei den bisher bekannten Geräten nicht gewährleistet werden kann.

### Darstellung der Erfindung

Aufgabe der vorliegenden Erfindung ist es, einen Inkubator der eingangs genannten Art so zu verbessern, daß die genannten Nachteile der bekannten derartigen Einrichtungen vermieden werden und daß insbesondere auf einfache Weise eine vorteilhafte Verwendung auch und gerade im Zusammenhang mit einem Handhabungsroboter möglich wird.

Diese Aufgabe wird gemäß der vorliegenden Erfindung bei einem Inkubator der genannten Art dadurch gelöst, daß die Trageböden Führungselemente für die Probenbehälter aufweisen, welche diese formschlüssig teilweise umgeben und hinsichtlich ihrer relativen Position festlegen und daß die Führungselemente frontseitig und rückseitig offene Einschubtröge für die Probenbehälter bilden, wobei front- und rückseitig je eine Tür vorgesehen ist, die im geschlossenen Zustand mit Anlageflächen beidseitig an den Probenbehältern anliegen und damit deren Lage in den Einschubtrögen festlegen. Damit kann also auch bei einer manuellen Einbringung der einzelnen Probenbehälter auf einfachste Weise eine genaue Positionierung derselben relativ zum Handhabungsroboter sichergestellt werden, ohne daß dabei aufwendige und langwierige Vorkehrungen dabei zu treffen wären, die den Ablauf beispielsweise eines Testzyklus nur unnötig verlängern würden.

Damit ist auch der Innenraum des Inkubators abgesehen von der üblichen frontseitigen Tür auch über eine rückseitige Tür zugänglich, was eine im Hinblick auf die Unfallsicherheit äußerst vorteilhafte komplette Trennung des Arbeitsbereiches eines beispielsweise über die Fronttür zugreifenden Handhabungsroboters vom Arbeitsbereich einer dann über die rückseitige Tür auf den Innenraum des Inkubators zugreifenden Bedienungsperson erlaubt. Nachdem von dieser die einzelnen Probenbehälter in die Einschubtröge gestellt wurden, kann einfach durch Schließen der Tür bzw. beider Türen eine genaue Lagefestlegung der Probenbehälter im Inkubator erfolgen, so daß der irgendwann anschließend nach dem Öffnen der ihm zugeordneten Tür zugreifende Handhabungsroboter die einzelnen Probenbehälter in genau definierter Position vorfindet.

Im letztgenannten Zusammenhang ist eine weitere Ausgestaltung der Erfindung von besonderem Vorteil, gemäß welcher die Anlageflächen (7) an der Seite einer der beiden Türen (6) elastisch ausgebildet sind, da damit ein sicheres und genaues Plazieren der Probenbehälter unter Ausgleich von Dimensionstoleranzen möglich ist. Die elastischen Anlageflächen bzw. -elemente sind bevorzugt natürlich auf der Seite des Handhabungsroboters vorzusehen, da dieser dann nach dem Öffnen der zugehörigen Tür die Probenbehälter auf alle Fälle in starr definierter Form vorfindet.

Nach einer anderen Weiterbildung der Erfindung ist vorgesehen, daß beide Türen über vorzugsweise pneumatische Betätigungselemente und eine gemeinsame Steuerung betätigbar sind, wobei das Öffnen einer Tür nur bei geschlossener anderer Tür möglich ist. Dies erhöht einerseits die Bedienungssicherheit des Inkubators weiter und sichert andererseits auch, daß der Handhabungsroboter beispielsweise nicht auf einen noch nicht durch die geschlossenen beiden Türen zentrierten Probenbehälter zugreifen kann, der dann unter Umständen nicht richtig gehalten wird und Störungen des Zyklus verursachen kann.

Die Trageböden sind in bevorzugter weiterer Ausgestaltung der Erfindung zusammen mit den Führungselementen in einem regalartigen Aufnahmegestell angeordnet, welches an Auflagebereichen am Gehäuse aufliegt und relativ zu diesem in Lage und/oder Ausrichtung verstellbar ist. Damit ist eine gewisse Justierung der gemeinsamen Lage aller Trageböden relativ zum Gehäuse des Inkubators bzw. damit auch zu einem Handhabungsroboter möglich, was den Ausgleich beispielsweise von Montage- und Aufstellungstoleranzen u.dgl. ermöglicht.

Das Gehäuse selbst kann im letztgenannten Zusammenhang in vorteilhafter Weise auch außen mit vorzugsweise verstellbaren Auflagen zur justierbaren Aufstellung und Befestigung an einer Unterlage ausgestattet sein, was weitergehende Berücksichtigung beispielsweise von Unebenheiten am Aufstellungsort u.dgl. ermöglicht.

Nach einer besonders bevorzugten weiteren Ausbildung der Erfindung ist vorgesehen, daß zur Aufnahme von Probenbehältern in Form von im wesentlichen quaderförmige Außenkontur aufweisenden Mikrotiterplatten die Führungselemente von über die Tiefe der Trageböden durchgehenden, im wesentlichen rechteckigen Führungsstreifen gebildet sind, deren Breite im wesentlichen dem seitlichen Abstand der Mikrotiterplatten entspricht und die auf den ansonsten ebenen Trageböden befestigt, vorzugsweise aufgeklebt, sind. Die genannten Mikrotiterplatten sind Aufbewahrungs- und Reaktionsgefäße für flüssige Proben. Breite und Länge (127.7 x 85.4 mm) der Mikrotiterplatten sind normiert; es gibt jedoch Ausführungen verschiedener Höhe. Sie können aus Polypropylen, Polystyrol, Polycarbonat oder anderen Kunststoffmaterialien gefertigt werden; können transparent oder opaque sein und mit oder ohne Deckel verwendet werden. Einige Formdetails der Mikrotiterplatten können zwischen den einzelnen Erzeugern variieren, wie z.B. die Höhe des Sockels; einige Hersteller schrägen zwei der vier Ecken der Mikrotiterplatte und des dazugehörigen Deckels ab, so daß diese Platten nur mit Deckeln desselben Erzeugers verwendet werden können. Bei anderen Mikrotiterplatten-Fabrikaten ist der Sockel in der Mitte der Platte erhöht. In die Mikrotiterplatten sind von oben Probenäpfchen eingelassen, die in der gebräuchlichsten Form in einer Matrix von 8 x 12 Näpfchen, das sind insgesamt 96 Näpfchen, angeordnet sind. Es gibt allerdings auch ähnliche Probenbehälter der gleichen Länge und Breite die nur 48, 24, 12 oder 6 Näpfchen enthalten, wobei die einzelnen Näpfchendurchmesser entsprechend größer sind.

Die häufigsten Anwendungsgebiete von Mikrotiterplatten liegen in der klinischen Diagnostik, wo sie z.B. beim ELISA-Verfahren (Enzyme-Linked Immuno Assay.) z.B. in Blutbanken verwendet werden; in Qualitätskontrollabors in der pharmazeutischen Industrie, wo sie zum Testen von Rohstoffen und Produkten auf größtmögliche Reinheit, z.B. im sogenannten Limulusverfahren verwendet werden; in der pharmazeutischen Forschung und Entwicklung, sowie in der Biotechnologie, wo sie in sogenannten Screening-Verfahren zur Anwendung kommen, bei denen aus einer großen Zahl von Testsubstanzen diejenigen mit dem gewünschten klinisch-therapeutischen Effekt gefunden werden können. Mikrotiterplatten finden aber auch Verwendung in der Lebensmittel- und Umweltchemie.

Der durch die Breite der Führungsstreifen bestimmte seitliche Abstand der einzelnen Mikrotiterplatten sowie auch der Abstand der einzelnen Trageböden ist nach unten im wesentlichen nur durch die im Hinblick auf die Handhabung durch den Roboter erforderliche Dimension begrenzt, so daß sich eine sehr hohe Packungsdichte und damit eine sehr gute Ausnutzung des Innenraumes des Inkubators ergibt.

### Kurze Beschreibung der Zeichnung

Die Erfindung wird im folgenden noch anhand der in der Zeichnung schematisch dargestellten Ausführungsbeispiele näher erläutert.
Fig. 1 zeigt dabei eine perspektivische Ansicht eines Inkubators nach der vorliegenden Erfindung,
Fig. 2 zeigt eine teilweise Draufsicht auf ein anderes Ausführungsbeispiel von vorne bei abgenommener Tür und
Fig. 3 zeigt einen teilweisen Schnitt entlang der Linie III/III in Fig. 2.

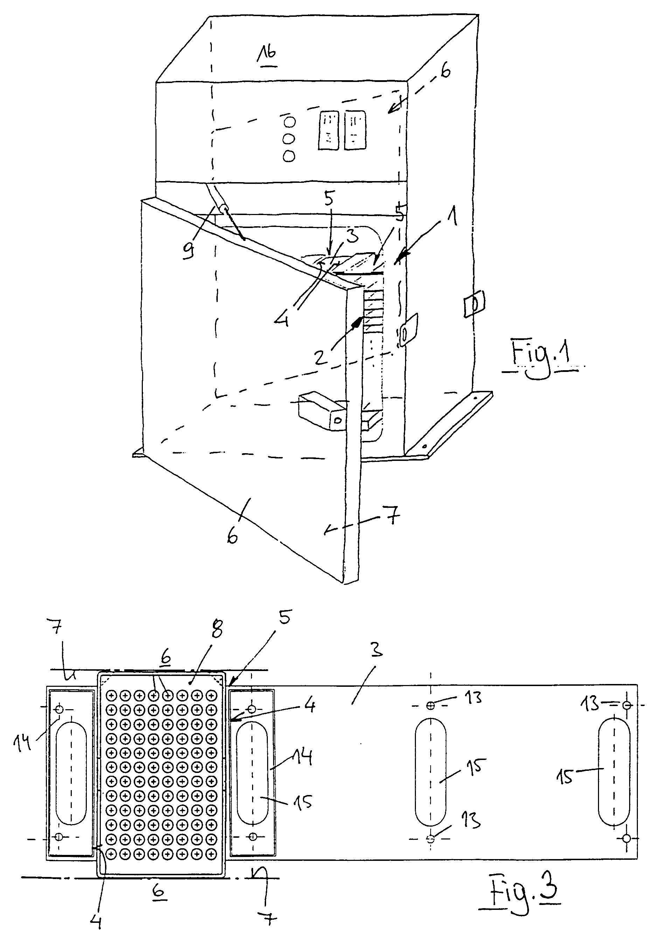

Der in Fig. 1 dargestellte Inkubator zur Aufnahme beispielsweise von lebenden Zellkulturen weist ein Gehäuse 1 und einen in diesem angeordneten Satz 2 von Trageböden 3 für hier nicht dargestellte, die Zellkulturen aufweisende Probenbehälter auf. Die Tragböden 3 haben Führungselemente 4 für die genannten Probenbehälter, welche diese im vorliegenden Fall in Form von frontseitig und rückseitig offenen Einschubtrögen 5 formschlüssig teilweise umgeben und hinsichtlich ihrer relativen Position im Inkubator festlegen.

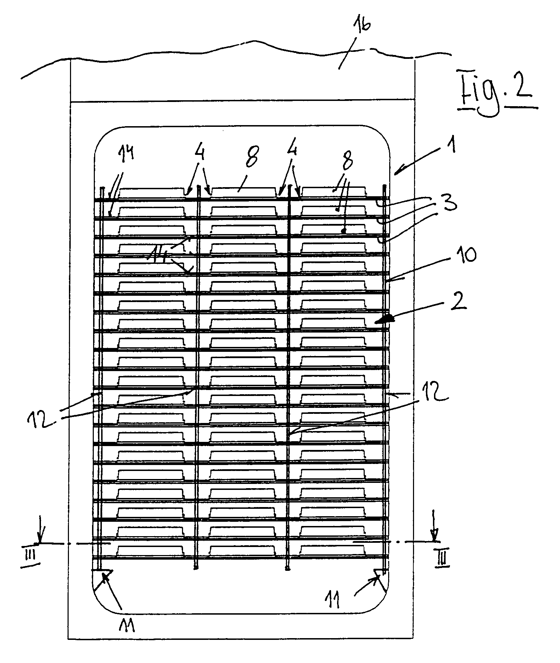

Front- und rückseitig ist hier je eine Tür 6 vorgesehen, die im geschlossenen Zustand (siehe auch Fig. 3) mit Anlageflächen 7 beidseitig an den Probenbehältern (8 in Fig. 3) anliegen und damit deren Lage auch in Einschubrichtung in den Einschubtrögen festlegen.

Beide Türen 6 sind über in Fig. 1 an der frontseitigen Tür 6 dargestellte, beispielsweise pneumatische Betätigungselemente 9 und eine hier nicht dargestellte gemeinsame Steuerung betätigbar, wobei die Steuerung vorzugsweise so erfolgt, daß das öffnen einer Tür 6 nur bei geschlossener anderer Tür 6 möglich ist. Auf diese Weise kann beispielsweise vorgesehen werden, daß über die frontseitige Tür ein hier ebenfalls nicht dargestellter Handhabungsroboter Zugriff auf den Innenraum des Inkubators bzw. die Probenbehälter 8 hat, während - bei geschlossener frontseitiger Tür - von der rückseitigen Tür 6 her eine Bedienungsperson auf die Probenbehälter 8 zugreifen und diese beispielsweise einlegen oder herausnehmen kann.

Die Trageböden 3 sind - wie insbesondere in Fig. 2 ersichtlich - zusammen mit den Führungselementen 4 in einem regalartigen Aufnahmegestell 10 angeordnet, welches an Auflagebereichen 11 am Gehäuse 1 aufliegt und auf hier nicht dargestellte Weise auch relativ zu diesem in Lage und/oder Ausrichtung verstellbar sein kann. Die einzelnen Trageböden 3 sind dabei über vertikale Verbindungen 12 zusammengesetzt, die entsprechende Löcher 13 in den Trageböden 3 durchsetzen und für den erforderlichen vertikalen Abstand zwischen den Trageböden 3 sorgen.

Zur hier dargestellten Aufnahme von Probenbehältern 8 in Form von im wesentlichen quaderförmige Außenkontur aufweisenden Mikrotiterplatten sind die Führungselemente 4 von über die Tiefe der Trageböden 3 durchgehenden, im wesentlichen rechteckigen Führungsstreifen 14 gebildet, deren seitliche Breite im wesentlichen dem seitlichen Abstand der Probenbehälter 8 bzw. Mikrotiterplatten entspricht und die auf den ansonsten ebenen Trageböden 3 befestigt, vorzugsweise aufgeklebt sind.

Um eine Zirkulation der Innenatmosphäre des Inkubators auch zwischen den eingelegten Probenbehältern 8 zu ermöglichen, sind gemäß Fig. 3 im Bereich der Führungsstreifen 14 langlochartige Ausnehmungen 15 in den Führungsstreifen 14 sowie in den Trageböden 3 vorgesehen, welche damit über die Höhe des Aufnahmegestells 10 durchgehende Strömungskanäle ermöglichen.

Der Vollständigkeit halber ist auch auf den in Fig. 1 und 2 ersichtlichen Aufbau 16 an der Oberseite des Gehäuses 1 zu verweisen, der hier nicht weiter dargestellte Elemente und Einrichtungen zur Regelung beispielsweise von Temperatur, Zusammensetzung und Feuchtigkeit der Innenatmosphäre des Inkubators enthält; weiters können hier auch Teile der Steuerlogik, der Türenbetätigung u.dgl. angeordnet sein, was eine vorteilhafte, zumindest teilweise Unabhängigkeit von einer Zentralsteuerung ermöglicht. Nicht dargestellt sind in diesem Zusammenhang Anschluß-, Versorgungs-, Steuerleitungen u.dgl.

Abgesehen von der dargestellten und beschriebenen Ausbildung des erfindungsgemäßen Inkubators zur Aufnahme von Mikrotiterplatten oder ähnlichen quaderförmigen Probenbehältern ist natürlich im Rahmen der Erfindung auch eine entsprechende Ausbildung beispielsweise für Petrischalen, Gewebekulturflaschen u.dgl. möglich, wozu jeweils im wesentlichen nur Form und Anordnung der Führungselemente entsprechend geändert werden müssen.

## Patentansprüche

1. Inkubator zur Aufnahme von Probenbehältern (8), mit einem frontseitig öffenbaren Gehäuse (1) und einem in diesem stapelartig, beabstandet übereinander angeordneten Satz von Trageböden (3) für jeweils mehrere bedarfsweise einzeln entfernbare Probenbehälter (8), dadurch gekennzeichnet, daß die Trageböden (3) Führungselemente (4) für die Probenbehälter (8) aufweisen, welche diese formschlüssig teilweise umgeben und hinsichtlich ihrer relativen Position festlegen und daß die Führungselemente (4) frontseitig und rückseitig offene Einschubtröge (5) für die Probenbehälter (8) bilden und daß front- und rückseitig je eine Tür (6) vorgesehen ist, die in geschlossenem Zustand mit Anlageflächen (7) beidseitig an den Probenbehältern (8) anliegen und damit deren Lage in den Einschubtrögen (5) festlegen.

2. Inkubator nach Anspruch 1, dadurch gekennzeichnet, daß die Anlageflächen (7) an der Seite einer der beiden Türen (6) elastisch ausgebildet sind.

3. Inkubator nach Anspruch 2, dadurch gekennzeichnet, daß beide Türen (6) über vorzugsweise pneumatische Betätigungselemente (9) und eine gemeinsame Steuerung betätigbar sind, wobei das Öffnen einer Tür (6) nur bei geschlossener anderer Tür (6) möglich ist.

4. Inkubator nach einem der Ansprüche 1 bis 3, dadurch gekennzeichnet, daß die Trageböden (3) zusammen mit den Führungselementen (4) in einem regalartigen Aufnahmegestell (10) angeordnet sind, welches an Auflagebereichen (11) am Gehäuse (1) aufliegt und relativ zu diesem in Lage und/oder Ausrichtung verstellbar ist.

5. Inkubator nach einem der Ansprüche 1 bis 4, dadurch gekennzeichnet, daß das Gehäuse (1) außen mit vorzugsweise verstellbaren Auflagen zur justierbaren Aufstellung und Befestigung an einer Unterlage ausgestattet ist.

6. Inkubator nach einem der Ansprüche 1 bis 5, dadurch gekennzeichnet, daß zur Aufnahme von Probenbehältern (8) in Form von im wesentlichen quaderförmige Außenkontur aufweisende Mikrotiterplatten die Führungselemente (4) von über die Tiefe der Trageböden (3) durchgehenden, im wesentlichen rechteckigen Führungsstreifen (14) gebildet sind, deren Breite im wesentlichen dem seitlichen Abstand der Mikrotiterplatten entspricht und die auf den ansonsten ebenen Trageböden (3) befestigt, vorzugsweise aufgeklebt, sind.

## Claims

1. Incubator for receiving sample holders (8), with a front-opening housing (1) and a set of supporting shelves (3) arranged spaced apart one above the other in a stack, each for a number of sample holders (8) which can be removed individually as required, characterised in that the supporting shelves (3) have guiding elements (4) for the sample holders (8) which enclose the latter mechanically at least partially and determine their relative position and that the guiding elements (4) form slide-in troughs (5) open at the front and back for the sample holders (8) and that a respective door (6) is provided both at the front and at the back, and each door has engaging faces (7) which in a closed condition engage the sample holders (8) on both sides and thereby determine their position in the troughs (5).

2. Incubator according to claim 1, characterised in that the engaging faces (7) are formed elastically on the side of one of the two doors (6).

3. Incubator according to claim 2, characterised in that both doors (6) are operated through preferably pneumatic actuating elements (9) and a common control, the opening of one door (6) being possible only with the other door (6) closed.

4. Incubator according to one of claims 1 to 3, characterised in that the supporting shelves (3) are arranged together with the guiding elements (4) in a bookcase-like receiving frame (10), which engages abutment regions (11) on the housing (1) and is adjustable relative to these in position and/or orientation.

5. Incubator according to one of claims 1 to 4, characterised in that the housing (1) is equipped externally with preferably adjustable mountings for adjustable placing and attachment on a base support.

6. Incubator according to one of claims 1 or 5, characterised in that for receiving sample holders (8) in the form of microtite plates of substantially rectangular external profile, the guiding elements (4) are formed by substantially rectangular guide strips (14) extending throughout the depth of the supporting shelves (3) and of which the breadth corresponds substantially to the lateral spacing of the microtite plates and which are secured, preferably by adhesion, to the otherwise flat supporting shelves (3).

## Revendications

1. Incubateur destiné à recevoir des récipients d'échantillons (8), muni d'un boîtier (1) susceptible d'être ouvert en face frontale et un jeu d'étagères (3) empilées et disposées à distance les uns au-dessus des autres et destinées chacune à recevoir plusieurs récipients d'échantillons (8) susceptibles, en cas de besoin, d'être enlevés individuellement, caractérisé en ce que les étagères (3) présentent, pour les récipients d'échantillons (8), des éléments de guidage (4) qui entourent partiellement ces récipients avec liaison par ajustement de forme et les fixent quant à leur position relative, et en ce que les éléments de guidage (4) constituent des bacs d'introduction (5) ouverts en face avant et en face arrière, pour les récipients d'échantillons (8), et en ce qu'une porte (6) est respectivement prévue en face avant et en face arrière, cette porte venant s'appuyer à l'état fermé, par des surfaces d'appui (7), des deux côtés sur le récipient d'échantillons (8) et fixant ainsi sa position dans les bacs d'introduction (5).

2. Incubateur selon la revendication 1, caractérisé en ce que les surfaces d'appui (7) sont réalisées élastiques sur la face de l'une des deux portes (6).

3. Incubateur selon la revendication 2, caractérisé en ce que les deux portes (6) sont actionnables, par l'intermédiaire d'éléments d'actionnement (9), de préférence pneumatiques, et d'une commande commune, l'ouverture d'une porte (6) n'étant possible que lorsque l'autre porte (6) est fermée.

4. Incubateur selon l'une des revendications 1 à 3, caractérisé en ce que les étagères (3) sont disposées, conjointement avec les éléments de guidage (4), dans un bâti de réception (10), en forme de rayonnage, qui repose sur des zones de pose (11) ménagées dans le boîtier (1) et qui sont réglables par rapport à celui-ci, en position et/ou en orientation.

5. Incubateur selon l'une des revendications 1 à 4, caractérisé en ce que le boîtier (1) est équipé extérieurement de supports de préférence réglables, pour la pose avec possibilité d'ajustement et la fixation sur une embase.

6. Incubateur selon l'une des revendications 1 à 5, caractérisé en ce que, pour recevoir les récipients d'échantillons (8) se présentant sous la forme de plaques de microtitrage présentant un contour extérieur sensiblement parallélépipédique, les éléments de guidage (4) sont constitués par des bandes de guidage (14) sensiblement rectangulaires, ininterrompues sur la profondeur des étagères (3) et dont la largeur correspond sensiblement à l'espacement latéral entre les plaques de microtitrage et qui sont fixées, de préférence collées, sur l'étagère (3), par ailleurs plane.
